# EUROPÄISCHE PATENTSCHRIFT

(11) **EP 1 509 127 B1**
(45) Veröffentlichungstag und Bekanntmachung des Hinweises auf die Patenterteilung: **20.04.2011**
(21) Anmeldenummer: 03740158.5
(22) Anmeldetag: 28.05.2003
(51) Int. Cl.: A61B 5/022

(54) **BLUTDRUCKMESSGERÄT**
BLOOD PRESSURE MANOMETER
TENSIOMETRE

(30) Priorität: 31.05.2002 DE 10224042
(43) Veröffentlichungstag der Anmeldung: 02.03.2005
(73) Patentinhaber: KAZ Europe SA, 1003 Lausanne (CH)
(72) Erfinder: FREUND, Dirk, 65779 Kelkheim (DE); HARTTMANN, Brigitte, 65527 Niedernhausen (DE); HECK, Ulrich, 47805 Krefeld (DE); HOLLINGER, Stefan, 61476 Kronberg (DE); RÖNNEBERG, Gerrit, 64289 Darmstadt (DE); SCHNAK, Fred, 61476 Kronberg (DE); SIMETH, Martin, 61462 Königstein (DE); WUNDER, Dieter, 63679 Schotten (DE); ZELLERMAYER, Siegfried, 60437 Frankfurt (DE)
(74) Vertreter: Grünecker, Kinkeldey, Stockmair & Schwanhäusser Anwaltssozietät
(86) Internationale Anmeldenummer: PCT/EP2003/005628
(87) Internationale Veröffentlichungsnummer: WO 2003/101290

(56) Entgegenhaltungen:
- EP-A- 0 744 155
- WO-A-01/50952
- DE-A- 3 426 183
- DE-A- 3 533 513
- GB-A- 862 875
- GB-A- 2 348 134
- US-A- 4 206 765
- US-A- 5 660 182
- US-A1- 2002 103 440

## Beschreibung

Die vorliegend Erfindung betrifft ein Blutdruckmeßgerät mit einem Gerätegehäuse, in dem Gerätebausteine wie ein Druckmesser, eine Anzeigeeinheit und dergleichen aufgenommen sind, einer mit dem Gehäuse verbundenen, um ein Körperglied legbaren Manschette, einem Manschettenspeicher, in den ein Manschettenabschnitt zur Weiteneinstellung der Manschette einziehbar ist, sowie einem Manschettenspanner, der den Manschettenabschnitt mit vorbestimmter Zugkraft in den Manschettenspeicher zieht.

Bei Blutdruckmeßgeräten ist es weit verbreitet, die Manschette mittels eines Klettverschlusses um ein Körperglied, insbesondere einen Oberarm oder ein Handgelenk, zu spannen. Zur Messung des arteriellen Blutdrucks wird eine in der Manschette vorgesehene Druckblase aufgepumpt, bis eine Arterie abgedrückt wird. Anschließend wird das Fluid aus der Druckblase langsam abgelassen, wobei durch den Puls eine Oszillation des Fluiddrucks in der Druckblase auftritt, anhand dessen sich z.B. der systolische und der diastolische Blutdruck bestimmen lassen. Dabei kommt es jedoch zu Meßwertschwankungen bzw. - fehlern, wenn die Manschette zu streng bzw. zu locker um das Körperglied gelegt wird. Letzteres läßt sich jedoch bei einem Anlegen der Manschette von Hand kaum vermeiden.

Es wurde daher bereits vorgeschlagen, die Manschette motorisch mit einer definierten Soll-Spannkraft um das Körperglied zu spannen. Die DE 198 59 392 A1 zeigt eine Spannvorrichtung für die Manschette eines Blutdruckmeßgerätes, bei der die Spannkraft automatisch auf eine vorgebbare Soll-Spannkraft begrenzt wird. Mittels eines Elektromotors werden zwei Wickelspulen angetrieben, auf die die Enden der Manschette aufgewickelt werden, wobei mittels eines Sensors die Kraft bestimmt und gesteuert wird, mit der die jeweiligen Manschettenabschnitte aufgewickelt werden und damit die Manschette um das Handgelenk gespannt wird. Diese bekannte Spannvorrichtung für Manschetten von Blutdruckmeßgeräten ist jedoch relativ aufwendig und erfordert eine eigene Sensorik, um die gewünschte Spannkraft zu erzielen. Zudem ist das An- und Ablegen der Manschette umständlich, da jedes Mal mittels des Stellmotors die Manschette auf- bzw. abgewickelt werden muß.

Weitere Blutdruckmeßgeräte nach dem Stand der Technik sind aus der DE 34 26 183 A, der GB 2 348 134 A, der DE 35 33 513 A, der US 4 206 765 A, der EP 0 744 155 A, der WO 01150952 A, der US 2002/103440 A1, der GB 862 875 A und der US 5 660 182 A bereits bekannt. Gemäß dem Stand der Technik wird zum Anlegen der Manschette der bekannten Blutdruckmeßgeräte eine Schlaufe gebildet, die groß genug ist, um das aufzunehmende Körperglied einzulegen und die anschließend festgespannt wird. Diese Handhabung hat sich als umständlich erwiesen.

Der vorliegenden Erfindung liegt daher die Aufgabe zugrunde, ein verbessertes Blutdruckmeßgerät der eingangs genannten Art zu schaffen, das Nachteile des Standes der Technik vermeidet und letzteren in vorteilhafter Weise weiterbildet. Vorzugsweise soll mit einfachen Mitteln sichergestellt werden, daß die Manschette reproduzierbar mit einer vorgebbaren Kraft um das Körperglied gespannt wird und ein einfaches An- und Ablegen der Manschette möglich ist.

Erfindungsgemäß wird diese Aufgabe durch ein Blutdruckmeßgerät gemäß Patentanspruch 1 gelöst. Bevorzugte Ausgestaltungen der Erfindung sind Gegenstand der Unteransprüche.

Erfindungsgemäß sind der Manschettenspeicher und der Manschettenspanner mit dem Gerätegehäuse in Verriegelungseingriff bringbar. Im Gegensatz zu Blutdruckmeßgeräten mit motorischen Manschettenspannem, die vom Gerätegehäuse getrennt an einem gegenüberliegenden Abschnitt der Manschette angeordnet sind, kann eine wesentlich kompaktere Anordnung erreicht werden. Zudem erhöht sich der Tragekomfort z.B. bei Langzeitmessungen, wenn die Manschette quasi nur an einem Umfangsabschnitt mit Anbauten, nämlich dem Gerätegehäuse versehen ist. Es versteht sich, daß die lösbare Anordnung des Manschettenspeichers und des Manschettenspanners am Gerätegehäuse unabhängig von der Ausbildung des Antriebs des Manschettenspanners besondere Vorteile besitzt.

Gemäß der Erfindung jedoch ist der Manschettenspeicher von dem Gerätegehäuse separat ausgebildet. Insbesondere kann er ein Ende der Manschette aufnehmen und als Verschlußteil ausgebildet sein, das mit dem Gerätegehäuse verriegelbar, vorzugsweise in das Gerätegehäuse einschiebbar ist. Um eine einfache Bedienung zu erreichen, kann eine formschlüssige Verriegelung insbesondere in Form einer Schnappverbindung vorgesehen sein. Die Ausbildung des Manschettenspeichers als Verschlußteil verbindet die Vorteile einer kompakten Bauweise mit hohem Anlege- und Ablegekomfort. Nach dem Einschieben des Manschettenspeichers in das Gerätegehäuse bilden diese beiden Komponenten eine Einheit. Andererseits erlaubt das Entriegeln und Abnehmen des Manschettenspeichers vom Gerätegehäuse ein vollständiges Öffnen der Manschette, so daß das Blutdruckmeßgerät einfach von dem Körperglied genommen werden kann. Es ist nicht nötig, die Manschette vollständig aus dem Manschettenspeicher auszuziehen, um etwa die Manschette über die Hand abstreifen zu können.

Um eine besonders einfache Handhabung zu erzielen, kann in Weiterbildung der Erfindung ein gemeinsamer Betätigungshebel für die Verriegelung des Manschettenspeichers am Gerätegehäuse und die Ausziehsperre vorgesehen sein. Um die Manschette zu öffnen, braucht lediglich der genannte Betätigungshebel gedrückt bzw. gezogen werden, der sodann gleichzeitig die Verrastung des Manschettenspeichers am Gerätegehäuse und die Ausziehsperre des Manschettenspanners löst. Hierdurch wird eine bequeme Ein-Hand-Bedienung ermöglicht.

Gemäß einem weiteren vorteilhaften Aspekt der vorliegenden Erfindung ist eine automatische Einschaltvorrichtung vorgesehen, die das Blutdruckmeßgerät nach dem Anlegen der Manschette an ein Körperglied automatisch einschaltet. Die Einschaltvorrichtung kann dabei grundsätzlich in Abhängigkeit verschiedener Einschaltbedingungen arbeiten, bei deren Vorliegen das Gerät eingeschaltet wird. So wäre es grundsätzlich möglich, die Spannkraft der Manschette zu erfassen und bei überschreiten eines vorgegebenen Wertes das Gerät einzuschalten. Vorzugsweise jedoch ist eine einfachere Einschaltbedingung maßgeblich. Die automatische Einschaltvorrichtung kann das Gerät insbesondere dann einschalten, wenn der Manschettenspeicher mit dem Gerätegehäuse in Eingriff gebracht wird. Hierzu kann an dem Gerätegehäuse ein Schalter zum Ein- und Ausschalten des Blutdruckmeßgerätes vorgesehen sein und der Manschettenspeicher bzw. der Manschettenspanner eine Betätigungseinrichtung aufweisen, die den Schalter beim Anlegen der Manschette in seine Einschaltstellung bringt. Es versteht sich, daß auch die Einschaltautomatik unabhängig von der Ausbildung des Antriebs des Manschettenspanners besondere Vorteile mit sich bringt, insbesondere eine völlig problemlose Handhabung des Geräts auch durch unkundige Bediener gestattet.

Um zu verhindern, daß das Blutdruckmeßgerät schon dann einschaltet, wenn die Manschette verschlossen wird, jedoch nicht um ein Körperglied gelegt ist, kann die Betätigungseinrichtung einen Unterbrecher aufweisen, der in Abhängigkeit der Einzugsstellung der Manschette in den Manschettenspeicher, insbesondere bei vollständig in den Speicher eingezogener Manschette, den Betrieb des Gerätes unterbricht. Der Unterbrecher kann in Abhängigkeit der auf die Wickelspule aufgewickelten Lagen arbeiten.

Vorzugsweise ist der Unterbrecher wirksam, wenn ein bestimmter Abschnitt der Manschette die Wickelspule erreicht hat. Insbesondere dann, wenn der aufwickelbare Manschettenabschnitt vollständig auf die Wickelspule aufgewickelt ist, wird die Betätigungseinrichtung unterbrochen.

Der Schalter am Gerätegehäuse kann zwei Kontakte aufweisen, die von zwei am Manschettenspeicher vorgesehenen Gegenkontakten geschlossen werden, wenn der Manschettenspeicher am Gerätegehäuse befestigt wird. Vorzugsweise ist an der Manschette ein Unterbrecherstück vorgesehen, das die Verbindung der beiden Gegenkontakte unterbricht, wenn die Manschette in ihre vollständig in den Manschettenspeicher eingezogene Stellung eingezogen ist. Dementsprechend schaltet die Einschakvorrichtung das Blutdruckmeßgerät automatisch nur dann ein, wenn einerseits die Manschette geschlossen ist, d.h. der Manschettenspeicher am Gerätegehäuse verriegelt ist, und andererseits die Manschette zumindest ein Stück weit ausgezogen ist, wie es immer dann der Fall ist, wenn die Manschette an einem Körperglied angelegt ist.

In Weiterbildung der Erfindung weist der Manschettenspanner also einen vorgespannten Federantrieb auf, der mit einer lösbaren Ausziehsperre zusammenwirkt, die ein ungewolltes Ausziehen der Manschette entgegen dem Federantrieb verhindert. Der Federantrieb bewirkt ein automatisches Straffziehen der Manschette mit zumindest einer individuell vorbestimmten Kraft, die individuell reproduzierbare Meßergebnisse sicherstellt. Dabei bewirkt der Federantrieb über eine gewisse Einziehlänge hinweg eine konstante Spannkraft, so daß unabhängig von der Dicke des jeweiligen Körperglieds die gewünschte Sollspannkraft erreicht wird. Alternativ kann die Spannkraft nur abhängig von der Einziehlänge und der Dicke des Körpergliedes individuell gleichbleibend bzw. vorbestimmt sein. Auch damit werden individuell reproduzierbare Meßergebnisse erreicht. Die Ausziehsperre hält dann die Manschette in der straffgezogenen Länge und verhindert, daß beim Aufblasen der der Manschette zugeordneten Druckblase die Manschette weiter gezogen wird. Im Gegensatz zu den bisher vorgeschlagenen Elektromotoren bzw. Pneumatikantrieben arbeitet der einfache Federantrieb ohne Fremdenergiezufuhr allein aufgrund seiner einmal vorgesehenen Vorspannung und aufgrund der im Federantrieb innewohnenden Federkraft durch das jeweilige Ausziehen und Entspannen der Manschette. Als Federantrieb sind alle nichtelektrischen Energiespeicher, wie auch eine Gummifeder oder Gasfeder, geeignet. Eine Stromzufuhr und entsprechende Batterien bzw. Akkus sind nicht nötig. Ebenfalls kann auf eine entsprechende Steuerung der Spannkraft durch Sensoren, Kraftaufnehmer etc. verzichtet werden. Andererseits erlaubt die erfindungsgemäße Manschettenspannerausbildung ein leichtes, rasches Ablegen der Manschette. Hierzu braucht lediglich die Ausziehsperre gelöst zu werden, so daß die Manschette entgegen der Federvorspannung aufgezogen oder aus dem Gerätegehäuse herausgenommen werden kann. Die Ausziehsperre kann durch ein Gesperr, Rasthaken, eine elektromagnetische Sperre oder eine Hemmung durch Einschieben des Manschettenspeichers in das Gehäuse (das Zusammenwirken von Gehäuse und Manschettenspeicher bewirkt eine Hemmung) ausgestaltet sein.

In Weiterbildung der Erfindung ist der Manschettenspanner als Wickelspule ausgebildet, die von dem Federantrieb angetrieben wird. Grundsätzlich wäre es auch möglich, einen Linearantrieb vorzusehen, um den Manschettenabschnitt in den Manschettenspeicher einzuziehen, vorzugsweise jedoch ist ein Drehantrieb mit einer Wickelspule vorgesehen, auf die der einzuziehende Manschettenabschnitt aufgewickelt wird. Die Wickelspule bildet einen Wickelspeicher, der sich durch seinen geringen Platzbedarf und die somit insgesamt erzielbare kompakte Anordnung auszeichnet. Die Ausziehsperre wirkt vorzugsweise unmittelbar auf die Wickelspule ein. Es könnte auch vorgesehen sein, daß die Ausziehsperre die Manschette selbst klemmt bzw. in anderer Weise festhält. Vorzugsweise jedoch verhindert die Ausziehsperre ein ungewolltes Zurückdrehen der Wickelspule. Sie kann vorteilhafterweise aus einer mit der Wickelspule verbundenen Rastverzahnung sowie zumindest einer zugehörigen Rastklinke bestehen.

Um eine kleinbauende Anordnung ohne weitere Übersetzungsstufen zu erreichen, kann der Federantrieb eine im Inneren der Wickelspule angeordnete Torsionsfeder besitzen, die auf einer drehfesten Wickelspulenachse aufgenommen ist, auf der drehbar die Wickelspule sitzt.

Diese und weitere Merkmale gehen außer aus den Ansprüchen auch aus der Beschreibung und den Zeichnungen hervor, wobei die einzelnen Merkmale jeweils für sich allein oder zu mehreren in Form von Unterkombinationen bei einer Ausführungsform der Erfindung und auf anderen Gebieten verwirklicht sein können. Ein Ausführungsbeispiel der Erfindung ist in der Zeichnung dargestellt und wird im folgenden näher erläutert. In den Zeichnungen zeigen:
- Figur 1: eine perspektivische Ansicht einer geöffneten Manschette eines Blutdruckmeßgerätes, die am Unterteil eines Gerätegehäuses befestigt ist, nach einer bevorzugten Ausführung der Erfindung,
- Figur 2: eine Draufsicht auf das Unterteil des Gerätegehäuses und einen am Ende der Manschette befestigten Manschettenspeicher aus Figur 1, der in das Unterteil des Gerätegehäuses einschiebbar ist,
- Figur 3: eine Draufsicht auf die Wickelspule eines Manschettenspanners im Inneren des Manschettenspeichers aus den vorhergehenden Figuren,
- Figur 4: einen Schnitt durch den Manschettenspeicher und den Manschettenspanner entlang der Linie A-A in Figur 3,
- Figur 5: eine Wickelspulenachse mit darauf angeordneter Torsionsfeder zur Vorspannung der Wickelspule aus Figur 3, und
- Figur 6: eine Draufsicht auf den Innenraum des Manschettenspeichers ähnlich Figur 3, wobei die Manschette in einer gänzlich eingezogenen Stellung gezeigt ist, in der ein auf der Manschette vorgesehenes Unterbrecherstück Einschaltkontakte unterbricht.

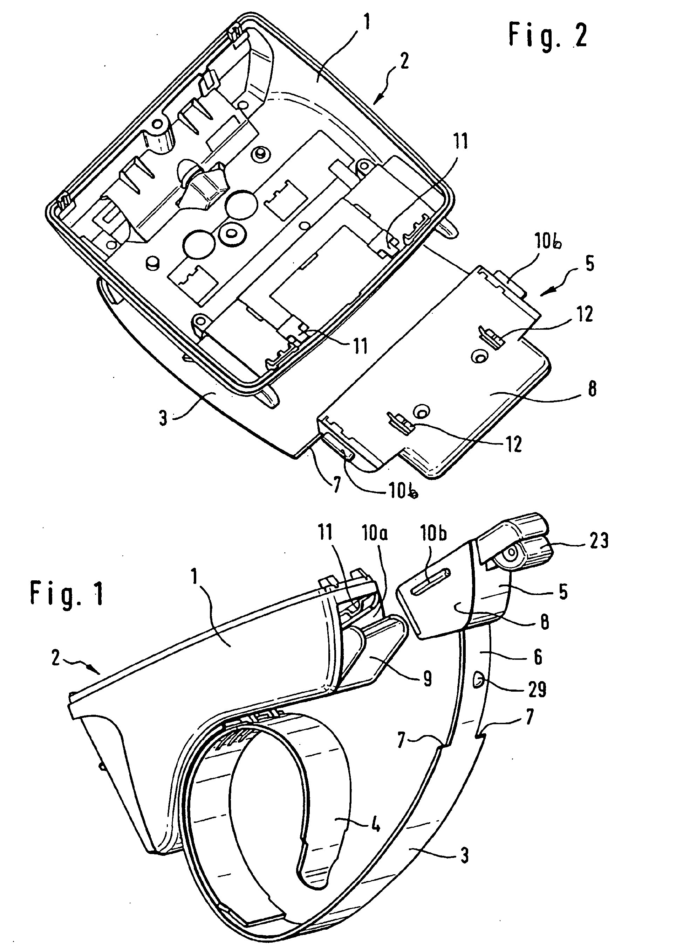

Aus Gründen der Übersichtlichkeit ist in Figur 1 ein Teil des Gerätegehäuses mit darin angeordneten Komponenten weggelassen. Gezeigt ist lediglich ein Unterteil 1 des Gerätegehäuses 2, an dem eine Manschette 3 befestigt ist, und zwar mittels eines Supportstücks 4. Im Gerätegehäuse 2 können in an sich für Handgelenks-Blutdruckmeßgeräte mit einer Messung nach dem oszillometrischen Prinzip bekannter Weise, ohne eigens gezeigt zu sein, eine Pumpe zum Aufpumpen einer der Manschette 3 zugeordneten Druckblase, ein Druckmesser, eine Auswerteeinheit sowie eine Anzeigeeinheit vorgesehen sein. Die Befestigung der Manschette 3 an dem Unterteil 1 umfaßt dabei eine Fluidkupplung, so daß von der Pumpe her die in der Manschette 3 angeordnete Druckblase (in den Figuren nicht dargestellt) aufgeblasen werden kann.

Das freie, d.h. nicht an dem Unterteil 1 befestigte Ende der Manschette 3 ist in einem Manschettenspeicher 5 aufgenommen und kann in diesen ein Stück weit eingezogen werden. Der Manschettenabschnitt 6, der in den Manschettenspeicher 5 eingezogen werden kann, wird von einem Anschlag 7 begrenzt, der eine vollständig eingezogene Stellung der Manschette 3 präzise definiert. Wie Figur 1 zeigt, kann der Anschlag 7 von einer stufenförmigen Verbreiterung der Manschette 3 gebildet sein, die beim Einziehen des Manschettenabschnitts 6 in den Manschettenspeicher 5 an dessen Gehäuse oder einem anderen geeigneten Gegenanschlagstück ansteht. Die Manschette 3 weist also ein flexibles - z.B. aus Kunststoff - etwa U-förmiges Supportstück 4 auf, das einerseits eine Befestigung der Manschette mit dem Gehäuseunterteil 1 und andererseits eine Vorfixierung des Blutdruckmeßgerätes beim Anlegen am Körperglied bzw. Handgelenk ermöglicht. Daneben weist die Manschette in üblicher Weise die Druckblase auf, die auf die zur Messung teils abgedrückte Arterie preßt. Außerdem ist in der Manschette ein Textilband vorgesehen, dessen eines Ende mit dem Supportstück befestigt und dessen anderes - schmäleres - Ende im Manschettenspeicher 5 aufwickelbar ist. Textilband, Supportstück und Druckblase sind von einem weiteren Textilstoff eingefaßt. In einer vorteilhaften Variante ist die Außenseite der Textilstoffeinfassung als in einem Endbereich schmäleres und aufwickelbares Textilband einstückig fortgeführt und ausgebildet, so daß ein extra Textilbandteil entfällt. Die Manschette ist frei von irgendwelchen Öffnungs- bzw. Trennmöglichkeiten, wie dies in an sich üblicher Weise durch einen Klettverschluß erfolgt.

Der Manschettenspeicher 5 besitzt ein Gehäuseteil 8 bzw. Verschlußteil 8, das passgenau in eine entsprechende Manschettenspeicherausnehmung 9 im Unterteil 1 des Gerätegehäuses 2 einschiebbar ist. Wie Figur 1 zeigt, ist die Manschettenspeicherausnehmung 9 an dem Unterteil 1 seitlich angeordnet, so daß der Manschettenspeicher 5 etwa in Umfangsrichtung eingeschoben werden kann. Eine Einschiebeführung in Form von Nuten 10a und passenden Vorsprüngen 10b an dem Unterteil 1 und dem Gehäuseteil 8 des Manschettenspeichers 5 zwingt die beiden Teile dabei in eine definierte Lage zueinander.

Der Manschettenspeicher 5 und das Unterteil 1 des Gerätegehäuses 2 können formschlüssig miteinander verriegelt werden. Die entsprechende Verriegelungsvorrichtung wird dabei von zwei Rasthaken 11, die federnd ausgebildet und in der Manschettenspeicherausnehmung 9 vorgesehen sind, sowie mit den Rasthaken 11 zusammenwirkende Rastausnehmungen 12 in der Oberseite des Gehäuseteils 8 des Manschettenspeichers 5 gebildet (vgl. Figuren 1 und 2). Beim Einschieben des Gehäuseteils 8 in die Manschettenspeicherausnehmung 9 werden die Rasthaken 11 zunächst zurückgedrückt. In der vollständig eingeschobenen Stellung können die Rasthaken 11 zurückfedern und in die Rastausnehmungen 12 einschnappen. Der Manschettenspeicher 5 bildet also ein Verschlußteil für die Manschette 3, das lösbar an dem Gerätegehäuse 2 verriegelt werden kann. Dies besitzt den Vorteil, daß beim Anlegen des Gerätes an das Handgelenk die Manschette mit einem Ende losgelöst vom Gerätegehäuse 2 frei um den Arm geführt werden und sodann am Gerätegehäuse 2 befestigt werden kann. Einerseits kann die Manschette also weiterhin geöffnet werden und andererseits sind die beiden miteinander zu verbindenen Teile, namentlich das Gerätegehäuse bzw Unterteil 1 und das Gehäuseteil 8 starr ausgeführt, so daß keines dieser Teile beim Verbinden flexibel ausweichen kann.

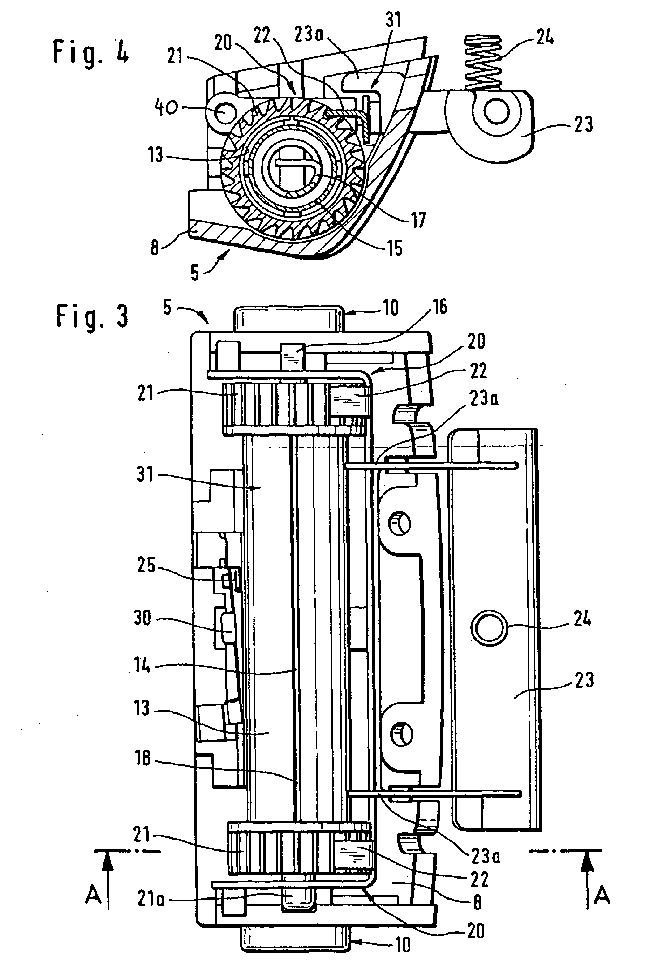

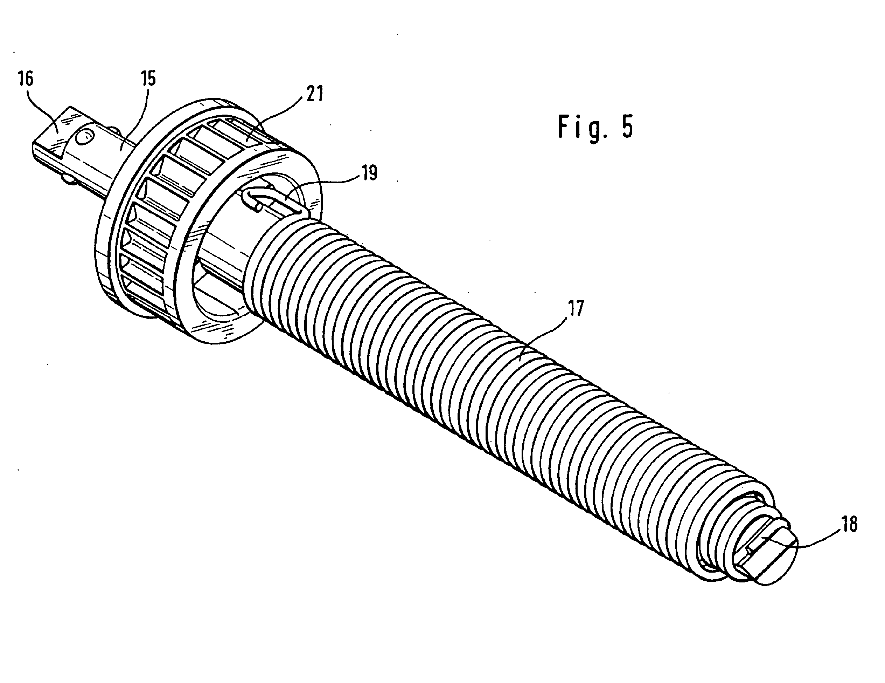

Im Inneren des Gehäuseteiles 8 des Manschettenspeichers 5 ist eine Wickelspule 13 angeordnet, auf der das freie Ende der Manschette 3 befestigt ist und aufgewickelt werden kann. Zur Befestigung der Manschette 3 kann die Wickelspule 13 einen Längsschlitz 14 aufweisen, in den das Ende der Manschette 3 eingeschoben werden kann. Die Wickelspule 13 selbst sitzt drehbar auf einer Wickelspulenachse 15, die drehfest in dem Gehäuseteil 8 gelagert ist. Wie Figur 3 zeigt, kann die Wickelspulenachse 15 mit Abflachungen an den Achsenenden in entsprechenden Ausnehmungen in dem Gehäuseteil 8 drehfest auf der einen Seite sitzen. Die Abflachung 16 an dem Ende der Wickelspulenachse 15 ist in Figur 5 zu sehen. Auf der anderen Seite sitzt der Zapfen 21a des Rastverzahnungrades 21 in der entsprechenden Ausnehmung des Gehäuseteils 8 als Gegenlager zur drehfesten Verbindung gegenüberliegend.

Die Wickelspule 13 ist gegenüber der Wickelspulenachse 15 vorgespannt, und zwar mittels einer Torsionsfeder 17, die als Federantrieb für die Wickelspule 13 dient. Wie Figur 5 zeigt, sitzt die Torsionsfeder 17 mit ihren schraubenförmigen Windungen auf der Wickelspulenachse 15. Ein erstes Federende 18 ist drehfest an der Wickelspulenachse 15 befestigt, während ein zweites Federende 19 drehfest an der Wickelspule 13 befestigt ist. Durch Drehen der Wickelspulenachse 15 bei gleichzeitigem Festhalten der Wickelspule 13 wird die Torsionsfeder 17 vorgespannt. In diesem Zustand wird die Wickelspule 13 in das Gehäuseteil 8 des Manschettenspeichers 5 eingesetzt, wobei die Abflachung 16 am Ende der Wickelspulenachse 15 in dem Gehäuseteil 8 des Manschettenspeichers 5 gefangen wird. Läßt man sodann die Wickelspule 13 los, zieht die vorgespannte Torsionsfeder 17 die Manschette 3 bis zum Anschlag 7 in das Gehäuseteil 8 des Manschettenspeichers 5 ein.

Ein ungewolltes Ausziehen der Manschette 3 verhindert eine Ausziehsperre 20. Wie Figur 3 zeigt, sind an beiden Enden der Wickelspule 13 Rastverzahnungen 21 vorgesehen, die Verzahnungshülsen bilden und mit der Wickelspule 13 drehfest verbunden sind. In die Rastverzahnungen 21 greifen Rastklinken 22 ein, die an einem Betätigungshebel 23 vorgesehen sind. Wie Figur 4 zeigt, ist der Betätigungshebel 23 schwenkbar um die Achse 40 an dem Gehäuseteil 8 gelagert und mittels einer Federeinrichtung 24 in seine die Rastverzahnungen 21 sperrende Stellung, d.h. gemäß Figur 4 nach unten vorgespannt. Die Vorspannung des Betätigungshebels 23 verhindert zum einen das Klappern der Rastklinken 22 und zum anderen ein ungewolltes Lösen der Ausziehsperre 20. Wie aus Fig. 3 ersichtlich, ist die Ausziehsperre in Einziehrichtung freigängig ausgebildet. Sie sperrt die Wickelspule 13 nur in Ausziehrichtung. In den Figuren 3 und 4 ist das zweite Widerlager zur Federeinrichtung 24, namentlich die Gehäuseoberschale des Betätigungshebels nicht dargestellt.

Wird der Betätigungshebel 23 entgegen seiner Federvorspannung gemäß Figur 4 nach oben gedrückt, werden die Rastklinken 22 von den Rastverzahnungen 21 außer Eingriff gebracht, so daß die Manschette 3 entgegen der Federvorspannung durch die Torsionsfeder 17 aus dem Manschettenspeicher 5 ausgezogen werden kann.

In einer bevorzugten Variante ist die Federeinrichtung 24 in entgegengesetzte Richtung wie oben ausgeführt drückend angeordnet, so daß der Betätigungshebel 23 in Figur 4 nach oben vorgespannt ist und die Rastverzahnung 21 entsperrt ist. Dadurch wird der Betätigungshebel 23 bereits durch die Federvorspannung nach oben gedrückt, es werden die Rastklinken 22 von der Rastverzahnung 21 außer Eingriff gebracht, so daß die Manschette 3 entgegen der Federvorspannung durch die Torsionsfeder 17 aus dem Manschettenspeicher 5 ausgezogen und mit der Vorspannung der Torsionsfeder 17 aufgewickelt werden kann. Demgemäß kann bei dieser Variante die Manschette unabhängig von einem Drücken des Betätigungshebels 23 auf und abgewickelt werden. Dies gilt jedoch, wie weiter unten fortgeführt, nur solange das Gehäuseteil 8 auch vom Unterteil 1 entriegelt ist, weil dann nicht die Federvorspannung der Rasthaken 11, die größer ist als die der Federeinrichtung 24 und zu dieser im verriegelten Zustand entgegengesetzt wirkt, für eine Sperrung der Manschette mittels der Rastverzahnung 21 sorgt.

Der Betätigungshebel 23 dient gleichzeitig dem Lösen der Verriegelung zwischen dem Manschettenspeicher 5 und dem Gerätegehäuse 2. Wie Figur 4 zeigt, besitzt der Betätigungshebel 23 Betätigungsabschnitte 23a in Form von nach oben vorspringenden Stegen, die unmittelbar unter den Rastausnehmungen 12 im Gehäuseteil 8 liegen. Wird der Betätigungshebel 23 gemäß Figur 4 nach oben gedrückt, drücken die Betätigungsabschnitte 23a die Rasthaken 11 in der Manschettenausnehmung 9 aus den Rastausnehmungen 12 im Gehäuseteil 8 bzw. verhindern ein Einschnappen der Rasthaken 11 in die Rastausnehmungen 12, so daß der Manschettenspeicher 5 aus der Manschettenspeicherausnehmung 9 herausgezogen werden kann. Dabei wird gleichzeitig die Ausziehsperre 20 entriegelt, so daß die Manschette 3 aus dem Manschettenspeicher 5 herausgezogen werden kann.

Die Rasthaken 11 sind an einem U- bzw. H-förmigen Blechbiegeteil angeformt und sind derart ausgebildet und angeordnet, daß sie mit einer gewissen Federvorspannung durch Öffnungen in die Manschettenspeicherausnehmung 9 in die Rastausnehmungen 12 des Gehäuseteils 8 bei verriegeltem Zustand hineinragen. Das Blechbiegeteil mit den Rasthaken 11 ist am Unterteil 1 befestigt.

Beim An- und Ablegen der Manschette kann dabei folgendermaßen vorgegangen werden:

Zunächst wird bei geöffneter Manschette diese auf das Handgelenk gelegt, wobei eine grobe Vorpositionierung durch das schalenförmige Supportstück 4 erreicht wird. Sodann wird die Manschette 3 mit ihrem freien Ende um das Handgelenk herumgeführt. Dabei wird vorteilhafterweise der Betätigungshebel 23 gegen das Oberteil des Gehäuses 8 des Manschettenspeichers 5 gedrückt, so daß die Ausziehsperre 20 gelöst wird. Hierdurch kann der Manschettenspeicher 5 einfach in die Manschettenspeicherausnehmung 9 am Gerätegehäuse 2 eingeschoben werden. Durch Loslassen des Betätigungshebels 23 können die Rasthaken 11 in die Rastausnehmungen 12 einschnappen, so daß die Manschette geschlossen ist. Gleichzeitig zieht die Torsionsfeder 17 die Manschette 3 straff.
Durch Loslassen des Betätigungshebels 23 ist auch die Ausziehsperre 20 wieder aktiviert worden. Auch beim Aufblasen der Manschette kann letztere nicht mehr aus dem Manschettenspeicher 5 herausgezogen werden.

Zum Lösen der Manschette 3 braucht lediglich der Betätigungshebel 23 gedrückt werden, um die Ausziehsperre 20 zu entriegeln und die Rasthaken 11 aus den Rastausnehmungen 12 herauszubewegen, so daß der Manschettenspeicher 5 aus dem Gerätegehäuse 2 herausgezogen werden kann.

Die bevorzugte Variante unterscheidet sich von der obigen Handhabung dadurch, daß der Betätigungshebel 23 nur zur Entriegelung der Rasthaken 11 aus den Rastausnehmungen 12 zum Herausbewegen des Manschettenspeichers 5 aus dem Gerätegehäuse 2 zu drücken ist, in den anderen Fällen jedoch nicht.

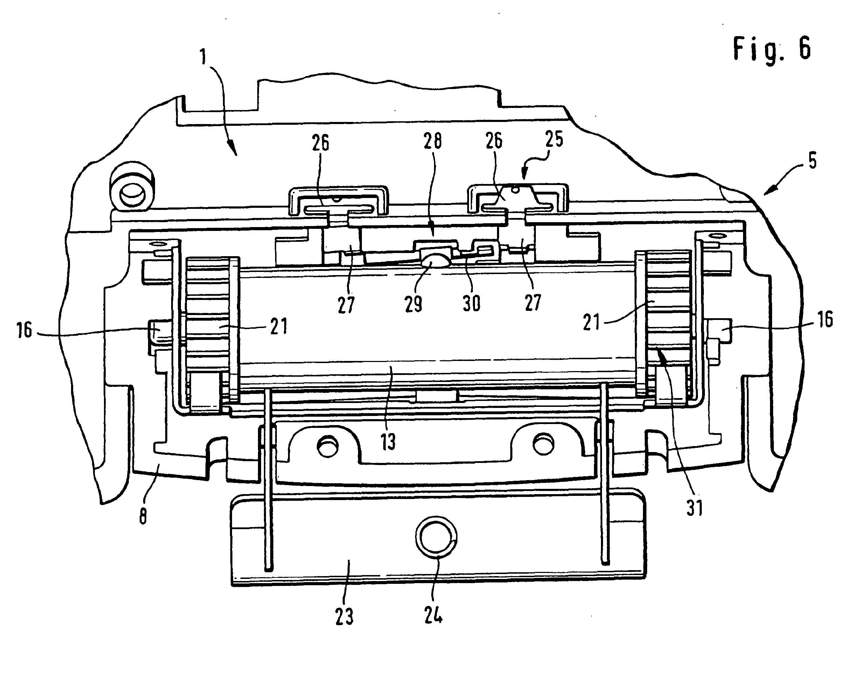

Vorteilhafterweise besitzt das Blutdruckmeßgerät eine automatische Einschaltvorrichtung 25. Wie am besten Figur 6 zeigt, sind in der Manschettenspeicherausnehmung 9 zwei Kontakte 26 vorgesehen, die elektrisch mit der Steuervorrichtung des Blutdruckmeßgerätes in dem Gerätegehäuse 2 verbunden sind. Gegenkontakte 27 sind an dem Gehäuseteil 8 des Manschettenspeichers 5 vorgesehen und kontaktieren die Kontakte 26, wenn das Gehäuseteil 8 des Manschettenspeichers 5 vollständig in die Manschettenspeicherausnehmung 9 eingeschoben ist. Die Kontakte sind zweckmäßigerweise entsprechend federnd ausgebildet. Sie können stirnseitig, d.h. in Einschubrichtung vorne an dem Gehäuseteil 8 angeordnet sein. Durch die Gegenkontakte 27 können die Kontakte 26 geschlossen, d.h. verbunden werden, wodurch sich das Gerät einschaltet.

Um jedoch zu verhindern, daß das Gerät eingeschaltet wird, wenn die Manschette geschlossen wird, ohne daß sie um ein Körperglied gelegt ist, ist ein Unterbrecher 29 vorgesehen, der das Schließen der Kontakte 26 bzw. die Verbindung der Gegenkontakte 27 in Abhängigkeit der auf die Wickelspule 13 aufgewickelten Manschettenlänge unterbricht. Wie die Figuren 1 und 6 zeigen, ist auf der Manschette 3 ein Unterbrecherstück 29 in Form eines vorspringenden Knopfes befestigt, der als Niet ausgebildet sein kann. Das Unterbrecherstück 29 ist dabei auf der Manschette 3 derart positioniert, daß es in der vollständig eingezogenen Stellung der Manschette im Bereich der Gegenkontakte 27 zu liegen kommt. Wie Figur 6 zeigt, sind die Gegenkontakte 27 miteinander durch einen Federbügel 30 verbunden. Wird die Manschette 3 vollständig in den Manschettenspeicher 5 eingezogen, drückt das Unterbrecherstück 29 die Federbügel 30 auf, d.h. die Gegenkontakte 27 geraten außer Verbindung. Sobald die Manschette 3 ein Stück weit ausgezogen wird, begibt sich das Unterbrecherstück 29 von dem Federbügel 30 weg, so daß die Gegenkontakte 27 miteinander verbunden werden. Hierdurch ist sichergestellt, daß die Einschaltautomatik das Gerät nur dann aktiviert, wenn die Manschette tatsächlich um ein Körperglied gelegt ist.

Ohne eigens gezeichnet zu sein, kann der Wickelspule eine Weitenmeßeinrichtung zugeordnet sein, die die abgerollte Länge der Manschette, d.h. die Manschettenweite und damit den Umfang des jeweiligen Körperglieds bestimmt. Die Bestimmung des Armumfangs durch die Rollmanschette kann in die Blutdruckberechnung durch die Auswerteeinheit des Geräts eingehen, um deren Genauigkeit weiter zu erhöhen oder auch der Unterscheidung verschiedener Benutzer zu dienen.

Das Blutdruckmeßgerät ist vorzugsweise derart ausgebildet, daß es mit der Unterseite des Gerätegehäuses 2 an der Schmalseite des Handgelenks angeordnet werden kann. Dementsprechend ist das Display mit seinen Anzeigefunktionen quer zur Unterarmerstreckung ausgerichtet, so daß die Anzeigefunktionen, wie Blutdruck und / oder Neigung des Gerätes ablesbar sind, sobald das Gerät mit dem Unterarm an den Brustkorb gelegt wird. Die Manschettespeicherausnehmung 9 ist hier am Gehäuse 2 zur Handrückenseite hin ausgerichtet. In einer Variante ist diese zur Handflächenseite hin ausgerichtet.

## Patentansprüche

1. Blutdruckmeßgerät mit einem Gerätegehäuse (2), in dem Gerätebausteine wie ein Druckmesser, eine Anzeigeeinheit und dergleichen aufgenommen sind, einer mit dem Gerätegehäuse (2) verbundenen, um ein Körperglied legbaren Manschette (3), einem Manschettenspeicher (5), in den ein Manschettenabschnitt (6) zur Weiteneinstellung der Manschette einziehbar ist, sowie einem Manschettenspanner (31), der den Manschettenabschnitt (6) mit vorbestimmter Zugkraft in den Manschettenspeicher (5) zieht,
**dadurch gekennzeichnet,**
**daß** der Manschettenspeicher (5) und der Manschettenspanner (31) von dem Gerätegehäuse (2) trennbar sind, wobei der Manschettenspeicher (5) und der Manschettenspanner (31) mit dem Gerätegehäuse (2) in lösbaren Verriegelungseingriff bringbar sind.

2. Blutdruckmeßgerät nach Anspruch 1, wobei der Manschettenspeicher (5) und der Manschettenspanner (31) in das Gerätegehäuse (2) integrierbar sind.

3. Blutdruckmeßgerät nach einem der vorhergehenden Ansprüche, wobei der Manschettenspeicher (5) ein Ende der Manschette (3) aufnimmt und als Verschlußteil ausgebildet ist, das mit dem Gerätegehäuse (2) verriegelbar ist.

4. Blutdruckmeßgerät nach Anspruch 3, wobei das Gerätegehäuse (2) eine Manschettenspeicherausnehmung (9) zur formschlüssigen Aufnahme des Verschlußteils (8) aufweist.

5. Blutdruckmeßgerät nach Anspruch 3 oder 4, wobei das Verschlußteil (8) und das Gerätegehäuse (2) mit korrespondierenden Einschiebeführungen (10a, 10b) ausgebildet sind, so daß das Verschlußteil (8) im Gerätegehäuse einschiebbar, insbesondere einschnappbar ist.

6. Blutdruckmeßgerät nach einem der vorhergehenden Ansprüche, wobei ein Betätigungsorgan (23), insbesondere -hebel vorgesehen ist, durch das gleichzeitig eine Verrastung des Manschettenspeichers (5) am Gerätegehäuse (2) und die Ausziehsperre (20) des Manschettenspanners (31) lösbar sind.

7. Blutdruckmeßgerät nach einem der vorhergehenden Ansprüche, wobei Federeinrichtungen (11, 24) vorgesehen sind, die derart zueinander wirkend angeordnet bzw. ausgebildet sind, daß bei Entriegelung des Manschettenspeichers (5) vom Gerätegehäuse (2) die Ausziehsperre (20) des Manschettenspanners (31) gelöst und bei Verriegelung des Manschettenspeichers (5) im Gerätegehäuse (2) die Ausziehsperre (20) des Manschettenspanners (31) ebenfalls verriegelt ist.

8. Blutdruckmeßgerät nach einem der vorhergehenden Ansprüche, wobei eine automatische Schaltvorrichtung (25) zum automatischen Ein- und/oder Ausschalten des Blutdruckmeßgerätes nach dem Anlegen der Manschette (3) an ein Körperglied vorgesehen ist.

9. Blutdruckmeßgerät nach Anspruch 8, wobei das Blutdruckmeßgerät durch die automatische Schaltvorrichtung (25) bei einem Öffnen der Manschette (3) während des Meßvorgangs als Not-Aus abschaltbar ist.

10. Blutdruckmeßgerät nach Anspruch 9, wobei das Blutdruckmeßgerät durch die automatische Schaltvorrichtung (25) nicht abschaltbar ist, soweit die Blutdruckmessung bereits abgeschlossen ist, so daß das Ergebnis der Messung angezeigt bleibt.

11. Blutdruckmeßgerät nach dem vorhergehenden Anspruch, wobei an dem Gerätegehäuse (2) ein Schalter (26) zum Ein- und Ausschalten des Blutdruckmeßgerätes vorgesehen ist und der Manschettenspeicher (5) und/oder der Manschettenspanner (31) eine Betätigungseinrichtung (27) aufweist, die den Schalter (26) beim Anlegen der Manschette in seine Einschaltstellung bringt.

12. Blutdruckmeßgerät nach dem vorhergehenden Anspruch, wobei die Betätigungseinrichtung (27) einen Unterbrecher (28, 30) aufweist, der in Abhängigkeit der Einzugsstellung der Manschette (3) In den Manschettenspeicher (5), insbesondere bei vollständig in den Manschettenspeicher eingezogener Manschette (3), den Betrieb des Gerätes unterbricht.

13. Blutdruckmeßgerät nach einem der beiden vorhergehenden Ansprüche, wobei der Schalter am Gerätegehäuse (2) zwei Kontakte (26) aufweist, die von zwei Gegenkontakten (27), die am Manschettenspeicher (5) vorgesehen sind, durch Befestigen des Manschettenspeichers (5) am Gerätegehäuse (2) schließbar sind, wobei vorzugsweise eine Verbindung der beiden Gegenkontakte (27) von einem Unterbrecherstück (29), das an der Manschette (3) befestigt ist, unterbrechbar ist, wenn die Manschette (3) in ihre vollständig in den Manschettenspeicher (5) eingezogene Stellung eingezogen wird.

14. Blutdruckmeßgerät nach einem der vorhergehenden Ansprüche, wobei die Manschette (3) frei von textilen Haftverbindern zur Weitenverstellung ausgebildet ist.

15. Blutdruckmeßgerät nach einem der vorhergehenden Ansprüche, wobei eine Meßeinrichtung zur Messung des Umfangs des Körperglieds, an das die Manschette jeweils angelegt ist, vorgesehen, insbesondere in den Manschettenspeicher (5) integriert ist.

16. Blutdruckmeßgerät nach Anspruch 1, wobei der Manschettenspanner (31) einen vorgespannten Federantrieb (17) aufweist, der mit einer lösbaren Ausziehsperre (20) zusammenwirkt, die ein ungewolltes Ausziehen der Manschette (3) entgegen dem Federantrieb (17) verhindert.

17. Blutdruckmeßgerät nach dem vorhergehenden Anspruch, wobei der Manschettenspanner (31) eine im Manschettenspeicher (5) angeordnete Wickelspule (13) aufweist, die vom Federantrieb (17) antreibbar ist, und die Ausziehsperre (20) auf die Wickelspule (13) einwirkt, vorzugsweise aus einer mit der Wickelspule (13) verbundenen Rastverzahnung (21) sowie zumindest einer zugehörigen Rastklinke (22) besteht.

18. Blutdruckmeßgerät nach dem vorhergehenden Anspruch, wobei der Federantrieb eine im Inneren der Wickelspule (13) angeordnete Torsionsfeder (17) aufweist, wobei vorzugsweise die Wckelspule (13) auf einer drehfesten Wickelspulenachse (15) sitzt, auf der die Torsionsfeder (17) angeordnet ist.

## Claims

1. Blood pressure measuring device with a device housing (2) in which device modules such as a manometer, a display unit and suchlike are accommodated, a cuff (3) connected to the device housing (2) which can be placed around a limb, a cuff storage device (5) into which a cuff portion (6) can be retracted in order to adjust the width of the cuff, as well as a cuff tensioning device (31), which draws the cuff portion (6) into the cuff storage device (5) with a predefined tensile force,
**characterised in that**
the cuff storage device (5) and the cuff tensioning device (31) are separable from the device housing (2), and the cuff storage device (5) and the cuff tensioning device (31) can be brought into a releasable locked engagement with he device housing (2).

2. Blood pressure measuring device as claimed in claim 1, in which the cuff storage device (5) and the cuff tensioning device (31) can be integrated in the device housing (2).

3. Blood measuring device as claimed in one of the preceding claims, in which the cuff storage device (5) accommodates one end of the cuff (3) and is provided in the form of a closure part which can be locked to the device housing (2).

4. Blood pressure measuring device as claimed in claim 3, in which the device housing (2) has a cuff storage recess (9) for a form locking accommodation of the closure part (8).

5. Blood pressure measuring device as claimed in claim 3 or 4, in which the closure part (8) and the device housing (2) are formed with corresponding insertion guides (10a, 10b) so that the closure part (8) can be inserted, in particular can be snap-fitted in the device housing.

6. Blood pressure measuring device as claimed in one of the preceding claims, in which an operating element (23), in particular an operating lever is provided by means of which a latching of the cuff storage device (5) onto the device housing (2) and the extraction lock (20) of the cuff tensioning device (31) are simultaneously releasable.

7. Blood pressure measuring device as claimed in one of the preceding claims, in which spring devices (11, 24) are provided, which are formed or arranged in such a co-operating manner that when the cuff storage device (5) is unlocked from the device housing (2), the extraction lock (20) of the cuff tensioning device (31) is released and when the cuff storage device (5) is locked in the device housing (2), the extraction lock (20) of the cuff tensioning device (31) is likewise locked.

8. Blood pressure measuring device as claimed in one of the preceding claims, in which an automatic switching device (25) is provided for automatically switching the blood pressure measuring device on and/or off after the cuff (3) has been placed on a limb.

9. Blood pressure measuring device as claimed in claim 8, in which the blood pressure measuring device can be switched off by the automatic switching device (25) as an emergency-off function in case of an opening of the cuff (3) during the measuring operation.

10. Blood pressure measuring device as claimed in claim 9, in which the blood pressure measuring device can not be switched off by the automatic switching device (25) if the blood pressure measurement has already been terminated so that the result of the measurement continues to be displayed.

11. Blood pressure measuring device as claimed in the preceding claim, in which a switch (26) is provided on the device housing (2) for switching the blood pressure measuring device on and off, and the cuff storage device (5) and/or the cuff tensioning device (31) has an operating mechanism (27) which moves the switch (26) into its switched-on position when the cuff is applied.

12. Blood pressure measuring device as claimed in the preceding claim, in which the operating mechanism (27) has an interrupter (28, 30) which interrupts operation of the device as a function of the retracting position of the cuff (3) into the cuff storage device (5), in particular when the cuff (3) has been fully retracted into the cuff storage device.

13. Blood pressure measuring device as claimed in one of the two preceding claims, in which the switch on the device housing (2) has two contacts (26) which can be closed by two mating contacts (27) provided on the cuff storage device (5) by attaching the cuff storage device (5) to the device housing (2), wherein preferably a connection of both mating contacts /27) can be interrupted by an interrupter device (29) which is attached to the cuff (3), when the cuff (3) is retracted in its position in which it is fully retracted into the cuff storage device (5).

14. Blood pressure measuring device as claimed in one of the preceding claims, in which the cuff (3) is free of any textile fasteners for adjusting the width.

15. Blood pressure measuring device as claimed in one of the preceding claims, in which a measuring device for measuring the circumference of the limb on which the cuff is placed, is provided and in particular is integrated in the cuff storage device (5).

16. Blood pressure measuring device as claimed in claim 1, in which the cuff tensioning device (31) has a pre-tensioned spring drive (17) which co-operates with a releasable extraction lock (20), which prevents any unintentional extraction of the cuff (3) against the spring drive (17).

17. Blood pressure measuring device as claimed in the preceding claim, in which the cuff tensioning device (31) has a spool (13) disposed in the cuff storage device (5) which can be driven by the spring drive (17), and the extraction lock (20) which acts on the spool (13), preferably comprises latch teeth (21) joined to the spool (13) and at least one co-operating latch pawl (22).

18. Blood pressure measuring device as claimed in the preceding claim, in which the spring drive has a torsion spring (17) arranged in the interior of the spool (13), and the spool (13) preferably sits on a non-rotating spool shaft (15) on which the torsion spring (17) is arranged.

## Revendications

1. Tensiomètre, comportant un boîtier (2), dans lequel sont logés des composants, tels qu'un manomètre, une unité d'affichage et tout composant similaire, une manchette (3), reliée au boîtier (2) et propre à être posée autour d'un membre, une réserve de manchette (5), dans laquelle peut être tiré un tronçon de manchette (6) pour le réglage de la largeur de la manchette, ainsi qu'un tendeur de manchette (31) qui tire le tronçon de manchette (6) avec une force de traction prédéterminée dans la réserve de manchette (5),
**caractérisé en ce que**
la réserve (5) et le tendeur (31) de la manchette peuvent être détachés du boîtier (2) du tensiomètre, la réserve (5) et le tendeur (31) de la manchette pouvant être amenés en prise de verrouillage amovible avec le boîtier (2) du tensiomètre.

2. Tensiomètre selon la revendication 1, dans lequel la réserve (5) et le tendeur (31) de la manchette peuvent être intégrés dans le boîtier (2) du tensiomètre.

3. Tensiomètre selon l'une quelconque des revendications précédentes, dans lequel la réserve de manchette (5) reçoit une extrémité de la manchette (3) et est réalisée sous la forme d'un élément de fermeture, qui peut être verrouillé avec le boîtier (2)du tensiomètre.

4. Tensiomètre selon la revendication 3, dans lequel le boîtier (2) comporte un évidement de la réserve de manchette (9) destiné à recevoir par conjugaison de forme l'élément de fermeture (8).

5. Tensiomètre selon la revendication 3 ou 4, dans lequel l'élément de fermeture (8) et le boîtier (2) du tensiomètre sont réalisés avec des guides d'insertion (10a, 10b) correspondants, de telle sorte que l'élément de fermeture (8) peut être inséré, en particulier enclenché, dans le boîtier du tensiomètre.

6. Tensiomètre selon l'une quelconque des revendications précédentes, dans lequel il est prévu un organe d'actionnement (23), en particulier un levier d'actionnement, qui permet de désolidariser simultanément un encliquetage de la réserve de manchette (5) an boîtier (2) du tensiomètre et l'élément anti-extraction (20) du tendeur de manchette (31).

7. Tensiomètre selon l'une quelconque des revendications précédentes, dans lequel sont prévus des dispositifs à ressort (11, 24) qui sont agencés ou réalisés de manière à agir l'un par rapport à l'autre, de telle sorte que lors du déverrouillage de la réserve de manchette (5) hors du boîtier (2) du tensiomètre, l'élément anti-extraction (20) du tendeur de manchette (31) est désolidarisé, et lors du verrouillage de la réserve de manchette (5) dans le boîtier (2) du tensiomètre, l'élément anti-extraction (20) du tendeur de manchette (31) est également verrouillé.

8. Tensiomètre selon l'une quelconque des revendications précédentes, dans lequel il est prévu un dispositif de commutation automatique (25) pour le branchement et/ou le débranchement automatiques du tensiomètre après la pose de la manchette (3) sur un membre.

9. Tensiomètre selon la revendication 8, dans lequel le tensiomètre peut être déconnecté sous la forme d'un arrêt d'urgence par le dispositif de commutation automatique (25) dans le cas d'une ouverture de la manchette (3) pendant le processus de mesure de la tension.

10. Tensiomètre selon la revendication 9, dans lequel le tensiomètre ne peut pas être déconnecté par le dispositif de commutation automatique (25) même si la mesure de la tension est déjà terminée, de telle sorte que le résultat de la mesure reste affiché.

11. Tensiomètre selon la revendication précédente, dans lequel il est prévu sur le boîtier (2) un commutateur (26) pour le branchement et le débranchement du tensiomètre, et la réserve de manchette (5) et/ou le tendeur de manchette (31) comporte(nt) un dispositif d'actionnement (27) qui amène le commutateur (26) dans sa position de branchement au moment de la pose de la manchette.

12. Tensiomètre selon la revendication précédente, dans lequel le dispositif d'actionnement (27) comporte un interrupteur (28, 30) qui, en fonction de la position d'introduction de la manchette (3) dans la réserve de manchette (5), en particulier lorsque la manchette (3) est insérée en totalité dans la réserve, interrompt le fonctionnement du tensiomètre.

13. Tensiomètre selon l'une des deux revendications précédentes, dans lequel le commutateur sur le boîtier (2) comporte deux contacts (26) qui, sous l'effet de la fixation de la réserve de manchette (5) sur le boîtier (2) du tensiomètre, peuvent être fermés par deux contacts complémentaires (27) prévus sur la réserve de manchette (5), sachant que, de préférence, une liaison des deux contacts complémentaires (27) peut être interrompue par un élément d'interruption (29) fixé sur la manchette (3), lorsque la manchette (3) est amenée dans sa position complètement introduite dans la réserve de manchette (5).

14. Tensiomètre selon l'une quelconque des revendications précédentes, dans lequel la manchette (3) est réalisée sans dispositif de serrage textile pour son réglage en largeur.

15. Tensiomètre selon l'une quelconque des revendications précédentes, dans lequel un dispositif de mesure, destiné à mesurer le pourtour du membre autour duquel la manchette doit être posée, est prévu, en particulier est intégré dans la réserve de manchette (5).

16. Tensiomètre selon la revendication 1, dans lequel le tendeur de manchette (31) comporte un entraînement à ressort (17) précontraint, qui coopère avec un élément anti-extraction (20) amovible, qui empêche une extraction non volontaire de la manchette (3) à l'encontre de l'entraînement à ressort (17).

17. Tensiomètre selon l'une quelconque des revendications précédentes, dans lequel le tendeur de manchette (31) comporte une bobine d'enroulement (13), qui est disposée dans la réserve de manchette (5) et qui,peut être actionnée par l'entraînement à ressort (17), et l'élément anti-extraction (20) qui agit sur la bobine d'enroulement (13) est formé de préférence par une denture de blocage (21), reliée à la bobine d'enroulement (13), et par au moins un cliquet de blocage (22) associé.

18. Tensiomètre selon l'une quelconque des revendications précédentes, dans lequel l'entraînement à ressort comporte un ressort de torsion (17) agencé à l'intérieur de la bobine d'enroulement (13), sachant que, de préférence, la bobine d'enroulement (13) est posée sur un axe (15) immobile en rotation, sur lequel est agencé le ressort de torsion (17).
